Europäisches Patentamt
European Patent Office
Office européen des brevets

(11) Publication number: **0 012 139**
**A1**

# (12) EUROPEAN PATENT APPLICATION


(21) Application number: 78300815.4

(22) Date of filing: 14.12.78

(51) Int. Cl.³: A 61 K 31/435, C 07 D 219/06

(43) Date of publication of application: 25.06.80 Bulletin 80/13

(84) Designated Contracting States: BE CH DE FR GB IT NL



(71) Applicant: AMERICAN CYANAMID COMPANY, Berdan Avenue, Wayne New Jersey 06904 (US)

(72) Inventor: Murdock, Keith Chadwick, 15 Birch Street, Pearl River, New York (US)
Inventor: Durr, Frederick Emil, 387 Jefferson, Ridgewood, New Jersey (US)
Inventor: Damiani, Martin Robert, 603, Franklin Turnpike, Allendale, New Jersey (US)

(74) Representative: Allam, Peter Clerk, TREGEAR, THIEMANN & BLEACH Enterprise House Isambard Brunel Road, Portsmouth, Hants PO1 2AN (GB)


## (54) Therapeutic agent for stimulating the immune response in a mammal.


(57) The present invention provides a therapeutic agent for stimulating the immune response in a mammal. The novel immunostimulant is a compound of the formula:

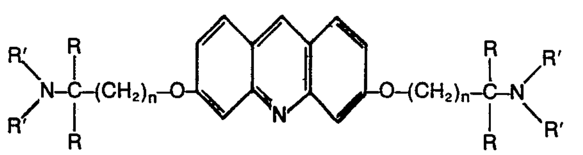

wherein n is the integer 1 or 2, R is hydrogen or methyl, and R' is alkyl having up to 4 carbon atoms, or a pharmaceutically acceptable acid-addition salt thereof.


EP 0 012 139 A1

0012139

TITLE: THERAPEUTIC AGENT FOR STIMULATING THE IMMUNE RESPONSE IN A MAMMAL

This invention relates to novel agents for stimulating the immune response in a mammal.

In U.S. Patent No. 3740403 there are disclosed 3,6-bis(dialkylaminoalkoxy) acridines and acid-addition salts thereof which may be represented by the following structural formula:

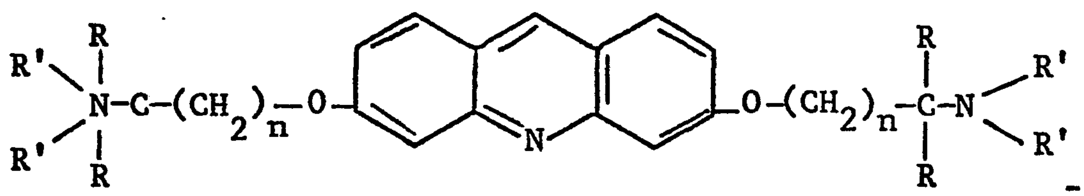

-(I)

wherein n is the integer 1 or 2; R is hydrogen or methyl; and R' is a lower alkyl group having up to 4 carbon atoms, such as methyl, ethyl, isopropyl and sec-butyl.

U.S. Patent No. 3740403 teaches that the compounds of Formula I possess antiviral activity as demonstrated in mice injected with $LD_{95}$ doses of Columbia SK virus. However, the U.S. patent does not teach the use of the Formula I compounds in any therapeutic method for the treatment of the human or animal body.

In accordance with the present invention it has now been discovered that compounds of Formula I, and pharmacologically acceptable acid-addition salts thereof, can be used to stimulate the immune response in a warm-blooded animal.

The compounds of Formula I are organic bases and thus are capable of forming acid-addition salts with a variety of organic and inorganic salt-forming reagents. Thus, acid-addition salts, formed by admixture of the organic free base with up to two equivalents of an acid, suitably in a neutral solvent, are formed with such acids as sulfuric, phosphoric, hydrochloric, hydrobromic, sulfamic, citric, malic, fumaric, tartaric, acetic, benzoic, gluconic, ascorbic, and related acids. The acid-addition salts of these compounds are, in general, crystalline solids relatively soluble in water, methanol and ethanol but relatively insoluble in non-polar organic solvents such as diethyl ether, benzene, toluene, and the like. For purposes of this invention, the free bases are equivalent to their non-toxic acid-addition salts.

The compounds of Formula I may be prepared by the process described in U.S. Patent No. 3740403 aforesaid.

The compounds of Formula I are active as immunostimulants when tested according to the following procedures:

(A) Rauscher leukemia virus

Rauscher leukemia virus is inoculated intraperitoneally into BALB/C mice. The virus inoculum is a 20% (W/V) spleen extract made from 21-day infected spleens of BALB/C mice. All mice are within a three gram weight range, with a minimum weight of 18 g., and all mice are of the same sex, usually male. Sheep red blood cells are injected intraperitoneally on the seventh day. There are 5 mice per test group. The test compound is administered orally on the sixth day as 0.5 ml. (in 0.2% Noble agar in saline) at a dose of 37.5 to 600 mg/kg of body weight, and again on the seventh and eighth day, in the same manner. On the fourteenth day the mice are weighed and bled from the retro-orbital sinus. The blood is pooled and the harvested serum is stored at $4^{\circ}C$ for 24 hours. Hemagglutinin tests are performed by standard procedures using the microtiter plate technique. Acceptable hemagglutinin titer for leukemic (immunosuppressed) mice is $\leq$ 1:128. Positive control compounds are Poly I:C (polyinosinic acid:-polycytidylic acid) administered intraperitoneally on days $^{+}6$, $^{+}7$ and $^{+}8$. Acceptable positive control hemagglutinin titers are 4-fold higher than the titers obtained in the leukemic control mice. The results of this test, using representative compounds of this invention, appear in Table I below.

## TABLE I

**Effect of Treatment on Antibody Response to Sheep Red Blood Cells in Leukemic Mice**

| Compound | Dose mg/kg | Hemagglutinin Titer* Test 1 | Test 2 | Test 3 | Test 4 | Test 5 | Test 6 |
|---|---|---|---|---|---|---|---|
| 3,6-Bis-(2-dimethylaminoethoxy)acridine | 100 | 128 | 128 | | | | |
| 3,6-Bis-(2-diethylamincethoxy)acridine trihydrochloride | 600 | | | | | | 256 |
| | 300 | | | | | | 128 |
| | 200 | | | | | 512 | |
| | 150 | | | | | | 128 |
| | 100 | | | 128 | 256 | 512 | |
| | 75 | | | | | | 128 |
| | 50 | | | | 128 | 128 | |
| | 37.5 | | | | | | 64 |
| 3,6-Bis-(3-dimethylaminopropoxy)acridine | 100 | 128 | 128 | | | | |
| 3,6-Bis-(2-dimethylamino-2-methylpropoxy)acridine trihydrochloride | 100 | 64 | | | | | |
| 3,6-Bis-(2-di-n-butylaminoethoxy)acridine trihydrochloride | 100 | 32 | | | | | |
| Poly I:C | 10 | 256 | 1024 | 512 | 256 | 1024 | 1024 |

Table I cont.

| Compound | Dose mg/kg | Hemagglutinin Titer* Test 1 | Test 2 | Test 3 | Test 4 | Test 5 | Test 6 |
|---|---|---|---|---|---|---|---|
| Non-infected, immunized (sheep red blood cell) control | | 1024 | 512 | 512 | 512 | 4096 | 2048 |
| Infected, immunized control** | | 8 | 32 | 32 | 32 | 128 | 32 |

* Reciprocal of serum dilution producing at least 50% agglutination of sheep red blood cells.
**Mice infected 7 days prior to injection of sheep red blood cells with Rauscher leukemia virus.

(B) Transplanted $C_3H$ mammary tumor

$C_3H$/HEB/J male mice were inoculated subcutaneously with five 2 mm. fragments of a transplantable $C_3H$ mammary tumor on day zero. Drugs were administered once every 7 days for 5 weeks, starting 14 days after tumor inoculation. When test drug (or the control, Corynebacterium parvum) was administered in combination with Cytoxan, it was given 4 days after the Cytoxan. Cytoxan and C. parvum were administered intraperitoneally; 3,6-bis(2-diethylaminoethoxy)acridine trihydrochloride was given orally. Tumor diameters were measured by means of a Vernier caliper. Test substances were considered active if tumor growth, as indicated by tumor diameter, was inhibited by 50% or more.

When 3,6-bis(2-diethylaminoethoxy)acridine trihydrochloride was administered alone at 300, 200 and 100 mg./kg. every week for 5 weeks, only the 200 mg./kg. dose level produced an inhibitory effect on mammary tumor growth compared to the untreated control. However, when combined with an ineffective dose of Cytroxan (60 mg./kg.), there appeared to be a therapeutic synergism at all dose levels of 3,6-bis(2-diethylaminoethoxy)acridine trihydrochloride with tumor growth being inhibited 50 - 65%. i.e., tumor diameter of treated mice averaging 107 - 160 $mm.^2$ compared to 316 $mm.^2$ for the untreated controls. The positive control, C. parvum, a killed preparation of bacteria demonstrated to be an immunomodulatory agent, produced an even greater inhibition of tumor growth when given in combination with Cytoxan. These results are set forth in Table II, below.

TABLE II

Effect of 3,6-Bis(2-diethylaminoethoxy)acridine trihydrochloride in Combination with Cytoxan on Mammary Tumor Growth in Mice

| Compound | Dose mg./kg. | + | Compound | Dose mg./kg. | Ave. tumor diameter ($mm.^2$) (weeks after tumor implantation) 3 | 4 | 5 | 6 | 7 | No. Survivors / No. Treated |
|---|---|---|---|---|---|---|---|---|---|---|
| Untreated Control | | | | | 22 | 73 | 149 | 218 | 316 | 7/8 |
| Cytoxan | 60 | | - | | 47 | 101 | 187 | 277 | 363 | 6/8 |
| 3,6-Bis(2--diethyl-aminoethoxy)acridine trihydrochloride | 300 | | - | | 61 | 98 | 220 | 375 | - | 1/8 |
| | 200 | | - | | 22 | 44 | 109 | 148 | 223 | 8/8 |
| | 300 | + | Cytoxan | 60 | 37 | 71 | 99 | 128 | 160 | 6/8 |
| | 200 | + | " | 60 | 32 | 66 | 82 | 115 | 135 | 7/8 |
| | 100 | + | " | 60 | 34 | 53 | 112 | 149 | 107 | 6/8 |
| C. parvum | 1.4mg. | + | Cytoxan | 60 | 18 | 17 | 35 | 41 | 63 | 8/8 |

The active compounds of the present invention are effective as immunostimulants (that is, they stimulate the immune response) when administered orally in amounts ranging from about 5 mg. to about 200 mg. per kilogram of body weight per day. A preferred dosage regimen for optimum results would be from about 5 kg. to about 50 mg. per kilogram of body weight per day, and such dosage units are employed that a total of from about 350 mg. to about 3.5 grams of the active compound for a subject of about 70 kg. of body weight are administered in a 24-hour period. This dosage regimen may be adjusted to provide the optimum therapeutic response. For example, several divided doses may be administered daily or the dose may be proportionally reduced as indicated by the exigencies of the therapeutic situation. A practical advantage of this invention is that the active compound may be administered in any convenient manner such as the oral or buccal routes or it may be incorporated directly in the diet.

The active compounds of the present invention may be orally administered, for example, with an inert diluent or with an assimilable edible carrier, or they may be enclosed in hard or soft shell gelatin capsules, or they may be compressed into tablets, or they may be incorporated directly with the food of the diet. For oral therapeutic administration, the active compounds may be incorporated with excipients and used in the form of tablets, troches, capsules, elixirs, suspensions, syrups, wafers, and the like. Such compositions and preparations should contain at least 0.1% of active compound. The

percentage of the composition and preparations may, of course, be varied and may conveniently be between about 2% to about 60% of the weight of the unit. The amount of active ingredient in such therapeutically useful compositions is such that a suitable dosage will be obtained. Preferred compositions or preparations according to the present invention are prepared so that an oral dosage unit form contains between about 50 and 250 milligrams of active compound.

The tablets, troches, pills, capsules and the like may also contain the following: a binder such as gum tragacanth, acacia, corn starch or gelatin; excipients such as dicalcium phosphate; a disintegrating agent such as corn starch, potato starch, alginic acid and the like a lubricant such as magnesium stearate; and a sweetening agent such as sucrose, lactose or saccharin may be added or a flavoring agent such as peppermint, oil of wintergreen, or cherry flavoring. When the dosage unit form is a capsule, it may contain, in addition to materials of the above type, a liquid carrier such as a fatty oil. Various other materials may be present as coatings or to otherwise modify the physical form of the dosage unit. For instance, tablets, pills, or capsules may be coated with shellac, sugar or both. A syrup or elixir may contain the active compound, sucrose as a sweetening agent, methyl and propylparabens as preservatives, a dye and flavoring such as cherry or orange flavor. Of course, any material used in preparing any dosage unit form should be pharmaceutically pure and substantially non-toxic in the amounts employed.

Examples of pharmaceutically useful compositions containing the present active compounds are now given by way of illustration.

## Example 1

### Preparation of 50 mg. Tablets

| Per Tablet | | Per 10,000 Tablets |
|---|---|---|
| 0.050 gm. | 3,6-bis(3-di-n-propylamino-3-methyl-n-butoxy)-acridine | 500 gm. |
| 0.080 gm. | Lactose..................... | 800 gm. |
| 0.010 gm. | Corn starch (for mix)....... | 100 gm. |
| 0.008 gm. | Corn starch (for paste)..... | 75 gm. |
| 0.148 gm. | | 1475 gm. |
| 0.002 gm. | Magnesium stearate (1%)..... | 15 mg. |
| 0.150 gm. | | 1490 gm. |

The 3,6-bis(3-di-n-propylamino-3-methyl-n-butoxy)-acridine, lactose and corn starch (for mix) are blended together. The corn starch (for paste) is suspended in 600 ml. of water and heated with stirring to form a paste. This paste is then used to granulate the mixed powders. Additional water is used if necessary. The wet granules are passed through a No. 8 hand screen and dried at 120°F. The dry granules are then passed through a No. 16 screen. The mixture is lubricated with 1% magnesium stearate and compressed into tablets in a suitable tableting machine.

## Example 2.

### Preparation of Oral Suspension

| Ingredient | Amount |
|---|---|
| 3,6-bis(3-di-n-butylamino-n-propoxy)acridine..................... | 500 mg. |

| Ingredient | Amount |
| --- | --- |
| Sorbitol solution (70. N.F.)............. | 40 ml. |
| Sodium benzoate.......................... | 150 mg. |
| Saccharin................................ | 10 mg. |
| Red dye.................................. | 10 mg. |
| Cherry flavor............................ | 50 mg. |
| Distilled water......qs......ad.......... | 100 ml. |

The sorbitol solution is added to 40 ml. of distilled water and the 3,6-bis(3-di-n-butylamino-n-propoxy)acridine is suspended therein. The saccharing, sodium benzoate, flavor and dye are added and dissolved. The volume is adjusted to 100 ml. with distilled water. Each ml. of syrup contains 5 mg. of active component.

CLAIMS:

1. For stimulating the immune response in a warm-blooded animal, a 3,6-<u>bis</u>-(dialkyl-aminoalkoxy) acridine compound of the formula:

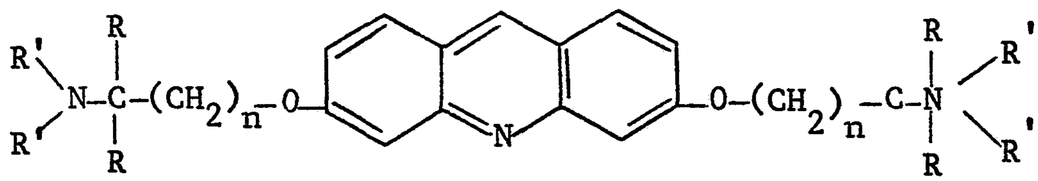

wherein n is the integer 1 or 2, R is hydrogen or methyl, and R' is alkyl having up to 4 carbon atoms, or a pharmaceutically acceptable acid-addition salts thereof.

2. The invention in accordance with Claim 1, wherein said compound is 3,6-bis(2-diethylaminoethoxy)-acridine trihydrochloride.

3. The invention in accordance with Claim 1, wherein said compound is 3,6-bis(3-dimethylaminopropoxy) acridine.

4. The invention in accordance with Claim 1, wherein said compound is 3,6-bis-(2-dimethylaminoethoxy) acridine.

5. The invention in accordance with Claim 1, wherein said compound is 3,6-bis-(2-dimethylamino-2-methylpropoxy)acridine trihydrochloride.

6. The invention in accordance with Claim 1, wherein said compound is 3,6-bis-(2-di-n-butylaminoethoxy) acridine trihydrochloride.

7. The invention in accordance with any preceding claim, wherein said compound is present together with a pharmaceutically acceptable carrier or diluent.

8. The invention in accordance with Claim 7, wherein the composition is presented in oral dosage unit form.

European Patent Office

# EUROPEAN SEARCH REPORT

0012139

Application number

EP 78 30 0815

| DOCUMENTS CONSIDERED TO BE RELEVANT | | | CLASSIFICATION OF THE APPLICATION (Int. Cl.²) |
|---|---|---|---|
| Category | Citation of document with indication, where appropriate, of relevant passages | Relevant to claim | |
| | | | A 61 K 31/435<br>C 07 D 219/06 |
| DX | US - A - 3 740 403 (K.C. MURDOCK)<br>* Column 1, lines 15-39, lines 48-60; column 2, lines 14-32; examples; claims *<br>---- | 1-8 | |
| | | | TECHNICAL FIELDS SEARCHED (Int.Cl.²) |
| | | | A 61 K 31/435<br>C 07 D 219/06 |
| | | | CATEGORY OF CITED DOCUMENTS |
| | | | X: particularly relevant<br>A: technological background<br>O: non-written disclosure<br>P: intermediate document<br>T: theory or principle underlying the invention<br>E: conflicting application<br>D: document cited in the application<br>L: citation for other reasons |
| | | | &: member of the same patent family, corresponding document |

✓ The present search report has been drawn up for all claims

| Place of search | Date of completion of the search | Examiner |
|---|---|---|
| The Hague | 05-07-1979 | NUYTS |